# EUROPEAN PATENT SPECIFICATION

(11) **EP 2 068 833 B1**
(45) Date of publication and mention of the grant of the patent: **09.01.2013**
(21) Application number: 07810734.9
(22) Date of filing: 25.07.2007
(51) Int. Cl.: A61K 9/127, A61K 47/48, A61P 29/00

(54) **PARENTERAL PREPARATIONS OF GI-SAFER PHOSPHOLIPID-ASSOCIATED ANTI-INFLAMMATORIES AND METHODS OF PREPARATION AND USE**
PARENTERALE ZUBEREITUNGEN PHOSPHOLIPID-ASSOZIIERTER GI-SICHERER ENTZÜNDUNGSHEMMER, ZUBEREITUNGSVERFAHREN UND VERWENDUNG
PRÉPARATIONS PARENTÉRALE D'ANTI-INFLAMMATOIRES ASSOCIÉS À DES PHOSPHOLIPIDES À TOXICITÉ GI RÉDUITE, ET PROCÉDÉS DE PRÉPARATION DE D'UTILISATION DE CELLES-CI

(30) Priority: 26.07.2006 US 833388 P
(43) Date of publication of application: 17.06.2009
(73) Proprietor: The Board of Regents of the University of Texas System, Austin, TX 78701 (US)
(72) Inventor: LICHTENBERGER, Lenard, M., Houston, TX 77096 (US); DIAL, Elizabeth, J., Houston, TX 77025 (US)
(74) Representative: Gates, Marie Christina Esther
(86) International application number: PCT/US2007/016666
(87) International publication number: WO 2008/013822

(56) References cited:
- EP-A- 1 205 181
- WO-A-2005/011600
- GB-A- 2 046 094
- US-A- 5 091 188
- US-A1- 2006 078 574

## Description

### BACKGROUND

This invention relates to the use of parenteral preparations of phospholipids and anti-inflammatory drugs, such as non-steroidal anti-inflammatory drugs ("NSAIDs") or cyclooxygenase-2 ("COX-2") inhibitors. In particular, this invention relates to phospholipid-associated anti-inflammatories that are useful for treating or preventing developmental conditions in low birth weight infants such as patent ductus arteriosus ("PDA") and retinopathy, with reduced gastrointestinal ("GI") injury.

NSAIDs are effective pain-relievers and anti-inflammatory agents that can be taken by mouth. However, in unconscious/unresponsive patients or in low birth weight neonates, oral dosing may not be possible and drugs must be injected parenterally. NSAIDs that are associated with phospholipids, such as phosphatidylcholine ("PC-NSAIDs") are new drugs that have fewer gastrointestinal side effects than regular NSAIDs and are safer for the patient when given chronically. Intravenously or intramuscularly administered NSAIDs can be used in the treatment of post-operative pain, but they create the risk of inducing GI ulceration and bleeding after surgery, and other concurrent damage to the GI membranes or layers. (Attridge et al.; Gabriel et al.; Wallace; Wolfe et al.). Because of solubility limitations, there are only a few NSAIDs that are approved for injections, and none of them are complexed to PC. In addition to post-operative pain, acute pain from vaso-occlusion due to sickle cell anemia may require hospitalization and parenteral pain management. Intravenous NSAID in the form of ketorolac has shown effective pain relief in many patients (Beiter et al.), but its chronic use is limited due to GI toxicity.

Additionally, as many as 20% of low birth weight infants suffer from Patent Ductus Arteriosus ("PDA"), or insufficient closure of the ductus arteriosus, resulting in blood of the neonate bypassing the lungs and causing inadequate oxygenation. Closure of the ductus arteriosus in full-term infants occurs normally and with no complications. However, in premature infants this closure may not occur and can lead to serious developmental complications. The recommended treatment to promote cloture of the duct is to administer intravenous indomethacin to inhibit prostaglandin synthesis in the vascular wall triggering the ductus closure. This treatment is not without risk as it has been reported that there is an association between the intravenous administration of indomethacin and other NSAIDs, and the development of another life-threatening condition called Spontaneous Intestinal Perforation ("SIP") which may be a form of a related disease entity called Necrotizing Enterocolitis ("NEC"), both of which have a 20% mortality rate (Attridge). Premature infants are also at risk for retinopathy due to retinal angiogenesis caused by hyperoxygenation in incubators. It may be possible to prevent this retinopathy of prematurity by parenterally administered ibuprofen which was reported to be effective in a murine model (Sharma).

A method to make PC-NSAIDs that are sterile and can be administered intravenously, intramuscularly, or by other parenteral routes with reduced risk of GI damage would be useful for all patients and particularly to mitigate post-operative and sickle cell pain, chronic neuropathic pain, and for low birth weight neonates suffering from insufficient closure of the ductus arteriosus and/or retinopathy.

### SUMMARY

The present invention pertains to parenteral preparations of phospholipid-associated anti-inflammatory drugs and their methods of preparation and use. In particular, the present invention pertains to parenteral preparations containing complexes of phospholipids and anti-inflammatory drugs such as NSAIDs or COX-2 inhibitors. Administration of the parenteral preparations is useful for the treatment and prevention of traumatic shock, post-operative pain, sickle cell pain, neuropathic pain, and the treatment of low birth weight infants suffering from Patent Ductus Arteriosus ("PDA") or retinopathy.

One aspect of the invention relates to a composition comprising a parenteral phospholipid and an anti-inflammatory pharmaceutical such as NSAIDs, COX-2 inhibitors or the like.

Another aspect of the invention is a method for making the parenteral preparations including the steps of contacting a phospholipid and an anti-inflammatory pharmaceutical in a heated polar solvent; removing the solvent, resuspending the anti-inflammatory-PC complex, and passing the composition through a membrane filter to produce a filter sterilized phospholipid-anti-inflammatory pharmaceutical preparation. An alternative method in preparing the sterile PC-associated anti-inflammatory for parenteral, oral, enteral or topical administration is to resuspend an injectable anti-inflammatory preparation, some of which may be commercially available, in a container containing PC as a dried powder or oil, followed by sonication or other means of agitation, and sterile filtration. Other means of sterilization include gamma irradiation, heat, chemical exposure, gas exposure, or a combination thereof.

A further aspect of the invention is a method for administering sterilized phospholipid-anti-inflammatory pharmaceutical compositions including the steps of orally administering, topically administering, intradermally administering, subcutaneously administering intramuscularly administering, intravenously administering, intra-arterially administering or directly administering into a tissue site an effective amount of the composition, where the administration can be a single administration, a periodic administration, a intermittent administration, or administration according to any administration protocol.

An additional aspect of the invention is a method of treating or preventing Patent Ductus Arteriosus ("PDA") and retinopathy in infants, and other uses of anti-inflammatory drugs in infants, by administering the compositions of this invention to the human or animal body by the above routes of administration or directly to the site of interest.

Background information pertaining to phospholipids and anti-inflammatory pharmaceuticals may be found in U.S. Patent Nos. 4,918,063, 5,043,329, 4,950,656;, 5,032,585, 5,763,422, and 5,955,451; U.S. Provisional Patent Application No. 60/256,711; U.S. Patent Application Serial No. 08/440,417; as well as International Patent Application Nos. PCT/US01/51605, PCT/US04/24807, and PCT/US05/36519,

A further aspect of the invention is a method of reducing the GI toxicity of anti-inflammatory drugs, and particularly NSAIDs, when administered by all routes of administration, to treat or prevent traumatic shock or post-operative pain, to treat or prevent sickle cell pain, to treat or prevent neuropathic pain, such as chronic spinal cord injury pain, to treat or prevent PDA and retinopathy of prematurity, and to reduce the incidence of GI injury, ulceration, bleeding, Spontaneous Intestinal Perforation ("SIP"), or Necrotizing Enterocolitis ("NEC") in a subject or patient, such as a low birth weight infant. The anti-inflammatory drugs that can be used in conjunction with phospholipids pursuant to the current invention include, but are not limited to, those listed in Table 1 below.

**Table 1**

| CHEMICAL CATEGORY | GENERIC NAME | TRADE NAME |
|---|---|---|
| Propionic acids | Fenoprofen calcium | Nalfon® |
| | Flurbiprofen | Ansaid® |
| | Suprofen | |
| | Benoxaprofen | |
| | Ibuprofen (prescription) | Motrin® |
| | Ibuprofen (200 mg OTC) | Nuprin®, Motrin IB® |
| | Ketoprofen | Orduis®, Oruvall® |
| | Naproxen | Naprosyn® |
| | Naproxen sodium | Aleve®, Anaprox®, Aflaxen® |
| | Oxaprozin | Daypro® |
| Acetic acids | Diclofenac sodium | Voltaren® |
| | Diclofenac potassium | Cataflam® |
| | Etodolac | Lodine® |
| | Indomethacin | Indocin® |
| | Ketorolac tromethamine (intramuscular) | Acular®**,** Toradol® |
| | Ketorolac (oral) | Toradol® |
| Ketones | Nabumetone | Relafen® |
| | Sulindac | Clinoril® |
| | Tolmetin sodium | Tolectin® |
| Fenamates | Meclofenamate sodium | Meclomen® |
| | Mefenamic acid | Ponstel® |
| Oxicams | Piroxicam | Feldene® |
| | Meloxicam | Mobic® |
| Salicylic acid | Diflunisal | Dolobid® |
| | Aspirin | |
| | Salsalate | Disalcid® |
| Pyrazolin acid | Oxyphenbutazone | Tandearil® |
| | Phenylbutazone | Butazolidin® |
| COX-2 inhibitor | Celecoxib | Celebrex® |
| | Rofecoxib | Vioxx® |
| | Valdecoxib | Bextra® |
| | Etoricoxib | Arcoxia® |
| | Lumiracoxib | Prexige® |

### BRIEF DESCRIPTION OF FIGURES

Figure 1 shows a graph comparing the analgesic effects of intravenously administered indomethacin ("INDO") and phosphatidylcholine ("PC")-associated INDO ("INDO-PC") in rats with Complete Freunds Adjuvant (CFA) joint inflammation. Indomethacin is given at 5mg/kg;

Figure 2 shows a graph comparing the incidence of the side effect of intestinal bleeding after intravenous administration of INDO and INDO-PC in rats that had been treated with the NO-synthase inhibitor, L-NAME (at a dose of 20 mg/kg i.p.) 1 hr before, 1 and 4 hrs after NSAID administration. Indomethacin is given at 5mg/kg;

Figure 3 shows a graph comparing the prostaglandin E₂ levels in inflamed tissue in rats with Complete Freunds Adjuvant (CFA) joint inflammation after intravenous administration of INDO and INDO-PC. Indomethacin is given at 5mg/kg;

Figure 4 shows a graph comparing the blood level of INDO and INDO-PC in rats with Complete Freunds Adjuvant (CFA) joint inflammation after intravenous administration of the drugs. Indomethacin is given at 5mg/kg;

Figure 5 shows a graph comparing the anti-inflammatory effects of subcutaneously administered INDO and INDO-PC in rats with Complete Freunds Adjuvant (CFA) joint inflammation. Indomethacin is given at 4mg/kg;

Figure 6 shows a graph comparing the incidence of side effects of intestinal adhesions and perforations after subcutaneous administration of INDO and INDO-PC in rats with Complete Freunds Adjuvant (CFA) joint inflammation. Indomethacin is given at 4mg/kg;

Figure 7 shows a graph comparing the prostaglandin E₂ levels in inflamed tissue in rats with Complete Freunds Adjuvant (CFA) joint inflammation after subcutaneous administration of INDO and INDO-PC. Indomethacin is given at 4mg/kg;

Figure 8 shows a graph comparing the side effect of gastric bleeding 4 hrs after intravenous administration of ketorolac and ketorolac-PC in rats. Ketorolac is given at 15mg/kg;

Figure 9 shows a graph comparing the side effect of intestinal bleeding 20 hrs after intravenous administration of diclofenac and diclofenac-PC in rats that had been treated with the NO-synthase inhibitor, L-NAME (at a dose of 20 mg/kg i.p.) 1 hr before, 1 and 4 hrs after UNSAID administration. Diclofenac is given at 30mg/kg.

### DETAILED DESCRIPTION OF PREFERRED EMBODIMENTS

This invention pertains to parenteral preparations of phospholipid-associated anti-inflammatory drugs, such as NSAIDs or COX-2 inhibitors. The PC-anti-inflammatory preparations are effective analgesics, anti-inflammatories, and anti-pyretics, as well as effective treatments for inducing the closure of the ductus arteriosus and preventing retinopathy in neonates, but with fewer gastrointestinal side effects. Although not wanting to be bound by any theory, our evidence that parenterally administered PL-associated NSAIDs have a reduced toxicity to the GI tract may be due to the unexpected likelihood that some or part of the PL remains attached to the NSAID during its secretion into the bile from the blood. In this way the NSAID that enters the GI lumen from the bile will have reduced toxicity to the GI mucosa due to it's association with PL.

In a first preferred embodiment, a parenteral pharmaceutical preparation is prepared by contacting a phospholipid and an anti-inflammatory pharmaceutical in a polar solvent, preferably at an elevated temperature in the range of about 30°C to about 60°C, cooling to room temperature if needed, removing the solvent by vacuum or by drying with an inert gas, resuspending the composition in an aqueous solution, and passing the composition through a membrane filter to produce a filter sterilized phospholipid-anti-inflammatory pharmaceutical preparation.

Examples of preferred phospholipids include phosphatidylcholine ("PC") and other zwitterionic phospholipids such as phosphatidylethanolamine, sphingomyelin and ceramides.

Examples of preferred anti-inflammatory pharmaceuticals include NSAIDs and COX-2 inhibitors. In additional preferred embodiments, the anti-inflammatory pharmaceutical is indomethacin, aspirin, ibuprofen, diclofenac, etodolac, ketorolac, celecoxib and any of the NSAIDs listed previously in Table 1.

Examples of preferred polar solvents include acetone, acetonitrile, dimethylformamide, dimethyl sulfoxide, methyl ethyl ketone, diethyl ether, and related solvents. The polar solvent is preferably utilized at an elevated temperature in the range of about 30°C to about 60°C, and most preferably at about 40°C. The solvent is preferably removed through evaporation or drying.

Examples of preferred aqueous solutions for resuspension include any suitable isotonic medium, such as sodium bicarbonate, saline, phosphate buffered saline, Ringer's lactate, dextrose, deoxycholate (at a weight/volume of about 0.05% to about 5%), and other IV solutions. Weight/volume as used herein is calculated by dividing the weight in grams of component A by 100ml of a solution.

The membrane filter through which the composition is passed to produce a sterile preparation is preferably one having a pore size in the range of about 0.22 µm to about 0.45 µm. Other methods of producing a sterile preparation include gamma irradiation, chemical exposure, gas treatment, heat, or a combination thereof.

In a second preferred embodiment, the parenteral preparation is prepared by mixing an injectable anti-inflammatory pharmaceutical with a phospholipid in the absence of an organic solvent, generally accompanied by agitating the composition through sonication or some other method, and finally passing the composition through a membrane filter for sterilization. The injectable anti-inflammatory pharmaceutical can be any commercially available injectable product. Examples of commercially available injectable NSAID preparations include: indomethacin sold under the name Indocin-IV® (Ovation Pharmaceuticals, Deerfield, IL); ketorolac tromethamine sold under the name Toradol® (Roche Laboratories, Nutley, NJ); diclofenac sodium sold under the name Votarol® (Novartis AG, Basel, Switzerland); and ibuprofen sold under the names NeoProfen® (Ovation Pharmaceuticals, Deerfield, IL) and Pedea® (Orphan Europe SARL, Paris, France). The phospholipid is preferably phosphatidylcholine in a dried powder or oil form.

Prior to use as a pharmaceutical product, the parenteral preparations are preferably adjusted to a physiological pH in the range of about 6.5 to about 8.

The sterile preparations can be administered through the steps of orally administering, topically administering, intradermally administering, subcutaneously administering, intramuscularly administering, intravenously administering, intra-arterially administering or directly administering into a tissue site an effective amount of the composition, where the administration can be a single administration, a periodic administration, a intermittent administration, or administration according to any suitable method of administration.

The effective dosage, based on the body weight of the subject, of sterile preparation effective for the treatment can range from about 0.1 mg/kg to about 100 mg/kg, and preferably from about. 1 mg/kg to about 20 mg/kg. The amount of PC in the sterile preparation can range from about 0.2 to about 400 mg/kg and preferably from about 2 mg/kg to about 40 mg/kg. The NSAID:PC weight ratio can range from 0.1:100 to 100:0.1 and is preferably from about 1:1 to about 1:3.

The parenteral preparations are used to treat Patent Ductus Arteriosus ("PDA") and prevent retinopathy in a subject such as a low birth weight infant. Preferable routes of administration for a subject are oral, enteral, or intravenous. Most preferred route of administration to an infant is intravenous administration. This treatment method has equivalent or enhanced therapeutic efficacy in inducing closure of the ductus arteriosus, with reduced toxicity to the GI tract. Treatment of infants having PDA with the sterile preparations rather than other anti-inflammatory compositions reduces the risk of the infant developing Spontaneous Intestinal Performations ("SIP") or Necrotizing Enterocolitis ("NEC").

The parenteral preparations may be used to prevent, treat, or ameliorate inflammation, pain, or fever with fewer gastrointestinal side effects. In particular, the sterile preparations can be used to treat or prevent traumatic shock and post-operative pain, pain from sickle cell anemia, and neuropathic pain, such as chronic pain from spinal cord injury.

### EXAMPLE 1. ANALGESIC AND ANTI-INFLAMMATORY ACTIVITY OF PARENTERAL PREPARATIONS OF PHOSPHOLIPID-ASSOCIATED ANTI-INFLAMMATORIES

Tests were performed to determine whether phosphatidylcholine ("PC") complexed with an NSAID possessed equivalent activity and side effects compared to an NSAID alone, when administered intravenously.

An UNSAID in the form of indomethacin (3 grams) was placed into a glass vial and dissolved in acetone. Phosphatidylcholine ("PC") (9 grams) was added to the vial and the combination was heated at 40°C for 10 minutes, during which time both the NSAID and PC dissolved. The acetone solvent was evaporated under nitrogen gas and the remaining indomethacin-PC was resuspended in 1.25% sodium bicarbonate by sonication in a bath sonicator. The pH of the suspension was adjusted to pH 7.4 and the lipid mixture was forced through a 0.22 µm membrane filter to sterilize it.

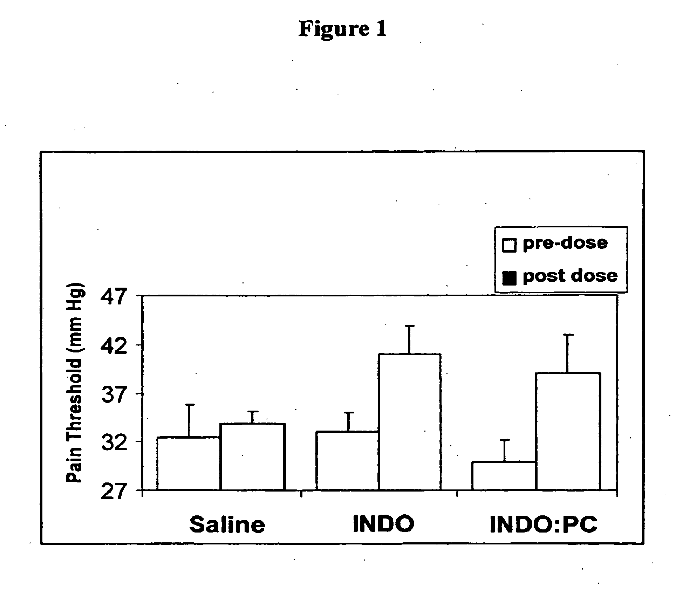

To test for analgesic activity, indomethacin ("INDO") and INDO-PC were administered intravenously to rats with a chemically-induced Complete Freunds Adjuvant ("CFA") paw inflammation, caused by the subcutaneous injection of 0.1 ml of CFA into the dorsal surface of the left hindpaw of a rat 4 days before. Saline was used as a control solution. Analgesia was assessed from the pain threshold which was measured by determination of the pressure which could be exerted on the inflamed paw before retraction by the animal, 30 minutes after IV administration of the test compounds. The results are shown in Figure 1. Both INDO and INDO-PC at a dose of 5 mg/kg demonstrated equivalent levels of analgesia as seen by an increased pain threshold.

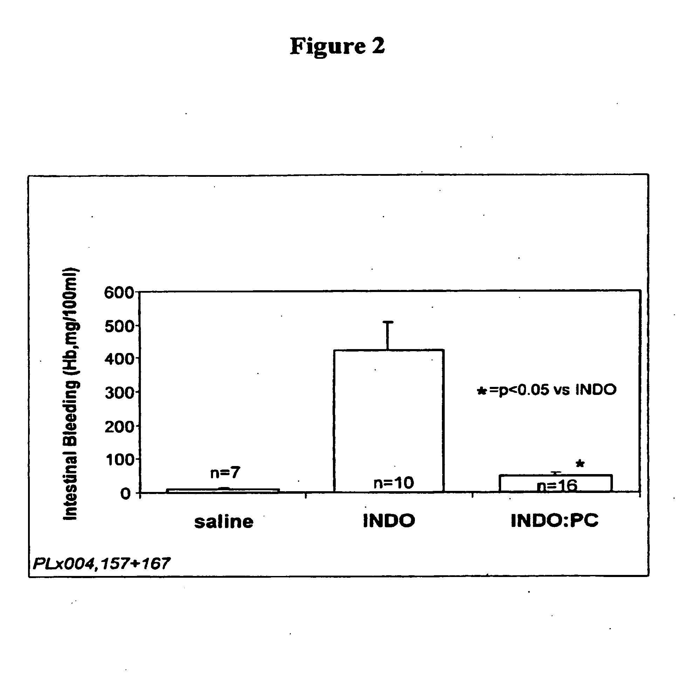

For determination of NSAID's side effects, rats were injected intraperitoneally with the nitric oxide ("NO") synthetase inhibitor N-tutroso-L-arginine methyl ester ("L-NAME") at a dose of 20 mg/kg, at 1 hr before and 1 and 3 hrs after being dosed with the tested NSAIDs, to increase their sensitivity to the GI toxic actions of the drugs. The rats were administered a dose of 10 mg/kg of intravenous INDO or INDO-PC and one day later, flushes of the small intestine were analyzed for the presence of hemoglobin as an indication of GI bleeding. Figure 2 shows that INDO-PC induced significantly less GI bleeding than INDO alone.

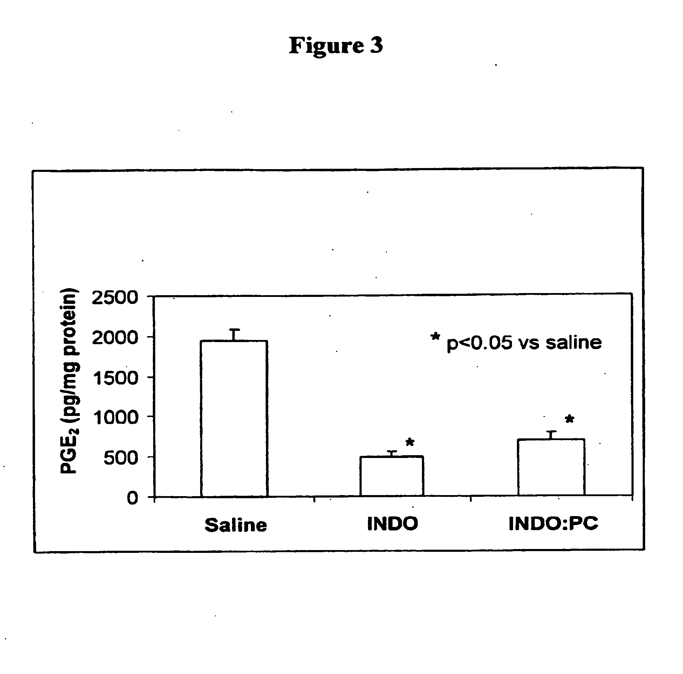

Another measure of drug activity was assessed by analysis of prostaglandin E₂ levels in inflamed tissue, which is typically lowered by indomethacin. In the same rats as shown in Figure 1, PGE₂ was measured in the inflamed paws 30 minutes after IV administration of the test compounds. It is seen in Figure 3 that both INDO and INDO-PC reduced PGE₂ levels of the synovial fluid of the inflamed joints in a significant and equivalent manner.

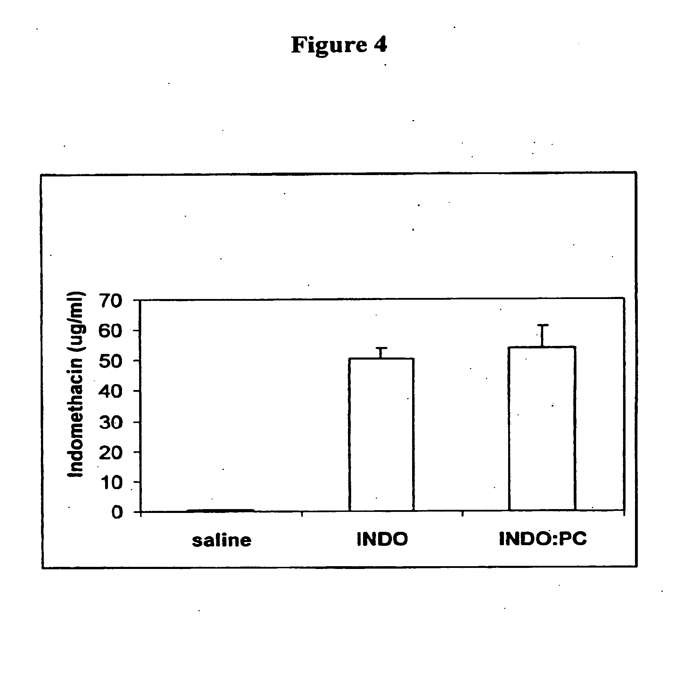

To determine if there was a difference in blood levels of the drugs, the same rats as shown in Figures 1 and 3 were bled at 30 minutes after drug administration, and the blood was analyzed for INDO content by high-pressure liquid chromatography ("HPLC"). Figure 4 shows that both INDO and INDO-PC had measurable blood levels of INDO and there was no difference between INDO and INDO-PC groups.

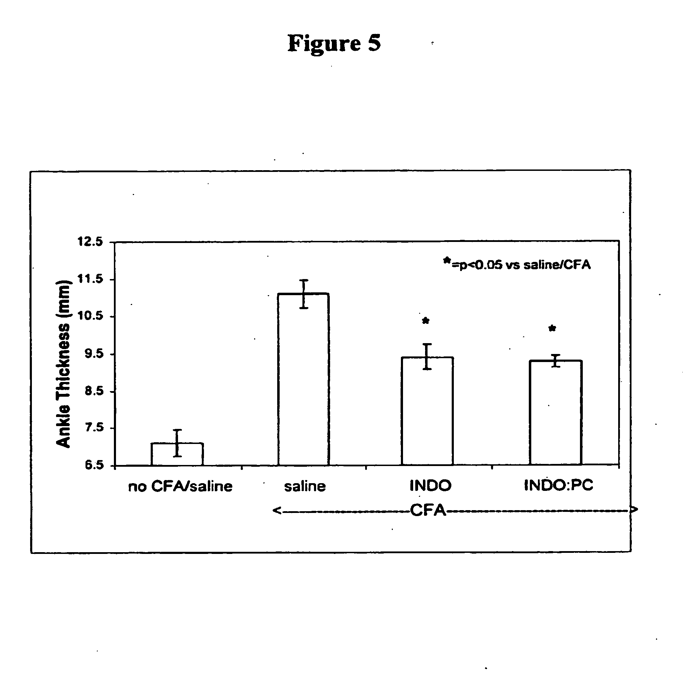

To test for anti-inflammatory activity after chronic drug administration, INDO and INDO-PC were administered subcutaneously for 4 days at 4 mg/kg to rats with a CFA induced paw inflammation. Inflammation was assessed by measurement of the thickness of the inflamed paw. The results in Figure 5 show that both INDO and INDO-PC significantly reduced paw swelling.

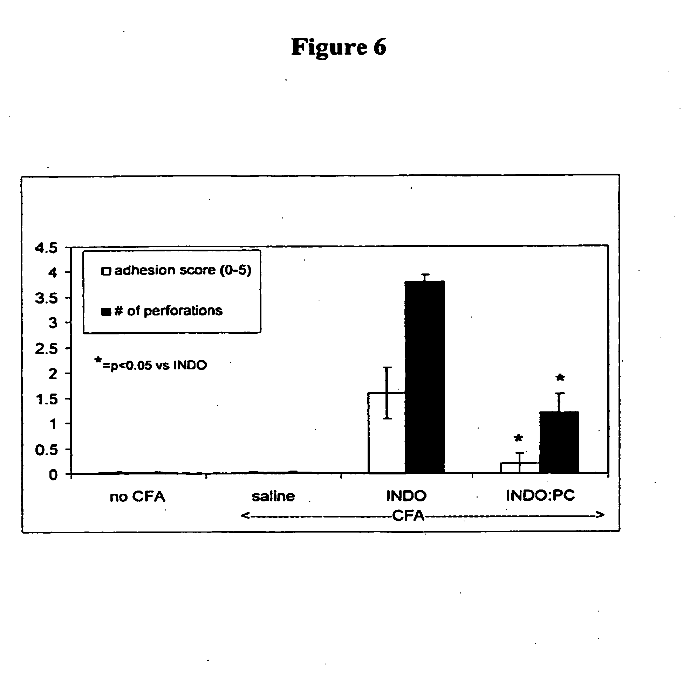

To assess GI side effects in the animals in Figure 5, measurements were made of the number of small intestinal adhesions and perforations. Figure 6 shows that INDO-PC gave significantly fewer intestinal adhesions and perforations than INDO.

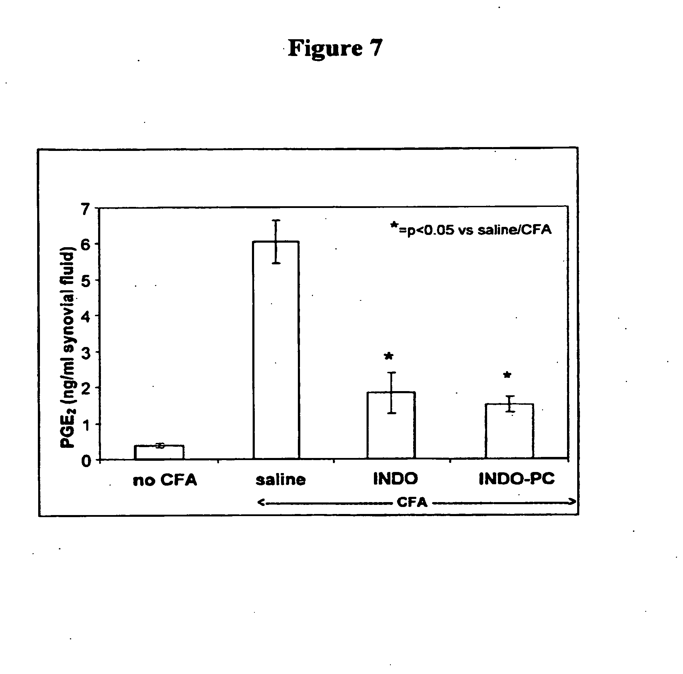

To assess anti-inflammatory activity by another means in chronically dosed animals, PGE₂ levels in paw synovial fluid of rats in Figure 5 was measured. The results in Figure 7 show that both INDO and INDO-PC significantly reduced inflammation-induced PGE₂ and in to a similar extent.

### EXAMPLE 2. REDUCED GI SIDE EFFECTS OF OTHER PC-NSAID COMPOUNDS

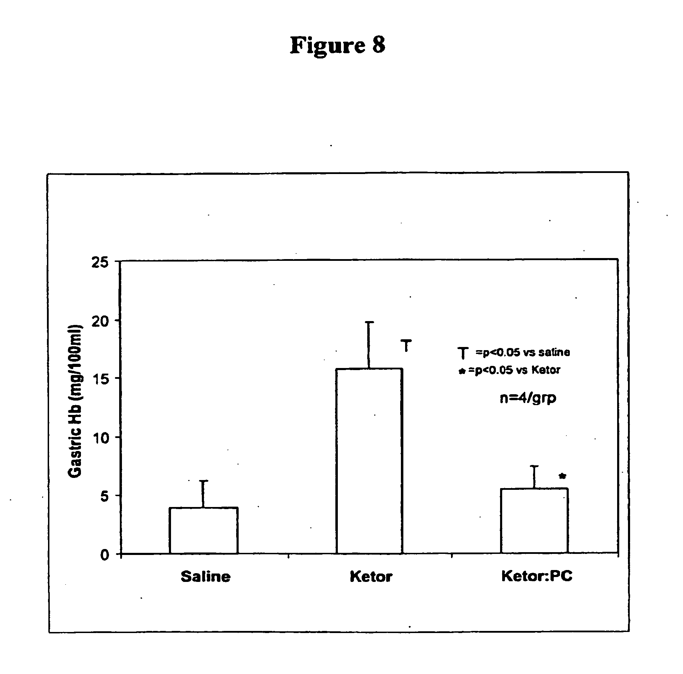

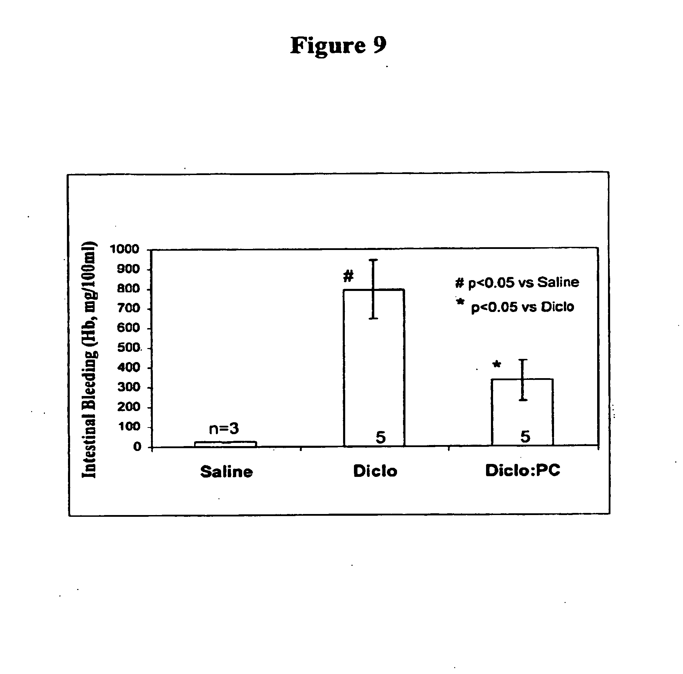

To show that other PC-NSAIDs exhibit reduced GI side effects, formulations of ketorolac-PC (1:2 (wt.:wt.)) and diclofenac-PC (1:2 (wt.:wt.)) were prepared. Rats were administered 15 mg/kg of intravenous ketorolac or ketorolac-PC; and 4 hours later flushes of the stomach were analyzed for the presence of hemoglobin as an indication of GI bleeding. Figure 8 shows that ketorolac-PC induced significantly less bleeding than ketorolac alone. Other rats were administered 30 mg/kg of intravenous diclofenac or diclofenac-PC in the L-NAME model described above. Flushes of the small intestine were analyzed for the presence of hemoglobin as an indication of GI bleeding. Figure 9 shows that diclofenac-PC induced significantly less GI bleeding than diclofenac alone.

### EXAMPLE 3. ANALGESIC EFFECTS OF IBUPROFEN-PC IN CHRONIC NEUROPATHIC PAIN

To show that ibuprofen-PC is effective at relieving the chronic neuropathic pain from spinal cord injury, rats are subjected to a controlled spinal injury and tested 6 weeks later after development of chronic pain. The injury consists of spinal T10 level impaction under anesthesia with an Infinite Horizon Impactor using 150 kDyne force for 1 second dwell time. After recovery for 6 weeks, animals are tested to confirm the presence of chronic pain by the Randall-Selitto test which consists of quantifying the withdrawal response to mechanical force applied to the hindpaw. Then ibuprofen or ibuprofen-PC at 1-100 mg/kg, iv, are administered and the rats tested again for pain 2 hours later.

Overall, these studies show that PC-NSAID formulations can be sterilized for intravenous, intra-arterial or direct administration into veins, arteries or tissues. The PC-NSAID formulation is equivalent in biological activity and blood level to the NSAID alone, but has less GI bleeding as a side effect, making it a safer drug..

### REFERENCES CITED

The following U.S. Patent documents and publications are relevant prior art.

### U.S. Patents

| **U.S. Patent No.** | **Issued to:** |
|---|---|
| 4,918,063 | Lichtenberger |
| 5,043,329 | Lichtenberger |
| 4,950,656 | Lichtenberger |
| 5,032,585 | Lichtenberger |
| 5,763,422 | Lichtenberger et al. |
| 5,955,451 | Lichtertberges et al. |

### Foreign Patent Documents

| | |
|---|---|
| PCCT/US01/51605 | Lichtenberger |
| PCT/US04/24807 | Lichtenberger |
| PCT/US05/36519 | Lichtenberger |

### Other Publications

Attridge, J.T., Clark, R, Walker, M.W., and Gordon, P.V. New insights into spontaneous intestinal perforation using a national data set: (1) SIP is associated with early indomethacin exposure. J. Perinatol., 2006; vol. 26: 93-99.
Beiter, J.L., Simon, H.K, Chambliss, C.R, Adamkiewicz, T., Sullivan, K. Intravenous ketorolac in the emergency department management of sickle cell pain and predictors of its effectiveness. Arch. Pediatr. Adolesc. Med. 2001; 155:496-500.
Lichtenberger LM, Wang Z-M, Romero JJ, Ulloa C, Perez JC, Giraud M-N, Barreto JC (1995). Non-steroidal anti-inflammatory drugs (NSAIDs) associate with zwitterionic phospholipids: Insight into the mechanism and reversal of NSAID-induced gastrointestinal injury. Nature Med 1: 154-158.
Gabriel, S.E., Jaakldmainen, L, and Bombardier, C. Risk for serious gastrointestinal complications related to use of nonsteroidal anti-inflammatory drugs: A meta-analysis. Ann Intern Med, 1991; vol. 115: 787-96.
Sharma, J., Barr, S.M., Geng, Y., Yun, Y., Higgins, R.D. Ibuprofen improves oxygen-induced retinopathy in a mouse model. Current Eye Research 2003; 27: 309-314.
Wallace, J.L. Nonsteroidal anti-inflammatory drugs and gastroenteropathy: the second hundred years. Gastroenterology 1997; vol. 112: 1000-1016.
Wolfe, P.A., Polhamus, C.D., Kubik, C., Robinson, A.B., Clement, D.J. Giant duodenal ulcers associated with post-operative use of ketorolac: report and three cases. Am. J. Gastroenterology 1994; vol. 89: 1110-1111.

## Claims

1. Use of an effective amount of a sterile preparation of a phospholipid-associated anti-inflammatory in the preparation of a medicament for treating Patent Ductus Arteriosus ("PDA") or retinopathy.

2. The use of claim 1, wherein the subject is a low birth weight infant.

3. The use of claim 1, wherein the anti-inflammatory is indomethacin, ibuprofen, or diclofenac.

4. The use of claim 1, wherein the phospholipid-associated anti-inflammatory is administered intravenously.

5. The use of claim 1, wherein the anti-inflammatory is a non-steroidal anti-inflammatory drug ("NSAID") and the administration of the NSAID is done intravenously.

6. Use of an effective amount of the parenteral preparation of a phospholipid-associated anti-inflammatory in the preparation of a medicament for inducing the closure of the ductus arteriosus.

7. The use of claim 6, wherein the subject is a low birth weight infant.

8. The use of claim 6, wherein the anti-inflammatory is indomethacin, ibuprofen, or diclofenac.

9. The use of claim 6, wherein the phospholipid-associated anti-inflammatory is administered intravenously.

10. The use of claim 6, wherein the anti-inflammatory is a non-steroidal anti-inflammatory drug ("NSAID") and the administration of the NSAID is done intravenously.

## Patentansprüche

1. Verwendung einer wirksamen Menge eines sterilen Präparats eines Phospholipid-assoziierten Antiphlogistikums bei der Herstellung eines Medikaments zur Behandlung von persistierendem Ductus arteriosus ("PDA") oder Retinopathie.

2. Verwendung nach Anspruch 1, wobei das Individuum ein Säugling mit niedrigem Geburtsgewicht ist.

3. Verwendung nach Anspruch 1, wobei das Antiphlogistikum Indomethacin, Ibuprofen oder Diclofenac ist.

4. Verwendung nach Anspruch 1, wobei das Phospholipid-assoziierte Antiphlogistikum intravenös verabreicht wird.

5. Verwendung nach Anspruch 1, wobei das Antiphlogistikum ein nichtsteroidaler entzündungshemmender Arzneistoff ("NSAID": non-steroidal anti-inflammatory drug) ist und die Verabreichung des NSAID intravenös erfolgt.

6. Verwendung einer wirksamen Menge des parenteralen Präparats eines Phospholipid-assoziierten Antiphlogistikums bei der Herstellung eines Medikaments, um den Verschluss des Ductus arteriosus zu induzieren.

7. Verwendung nach Anspruch 6, wobei das Individuum ein Säugling mit niedrigem Geburtsgewicht ist.

8. Verwendung nach Anspruch 6, wobei das Antiphlogistikum Indomethacin, Ibuprofen oder Diclofenac ist.

9. Verwendung nach Anspruch 6, wobei das Phospholipid-assoziierte Antiphlogistikum intravenös verabreicht wird.

10. Verwendung nach Anspruch 6, wobei das Antiphlogistikum ein nichtsteroidaler entzündungshemmender Arzneistoff ("NSAID": non-steroidal anti-inflammatory drug) ist und die Verabreichung des NSAID intravenös erfolgt.

## Revendications

1. Utilisation d'une quantité efficace d'une préparation stérile d'un anti-inflammatoire associé à un phospholipide dans la préparation d'un médicament destiné au traitement de la persistance du canal artériel (PDA) ou de la rétinopathie.

2. Utilisation selon la revendication 1, dans laquelle le sujet est un nourrisson de faible poids de naissance.

3. Utilisation selon la revendication 1, dans laquelle l'anti-inflammatoire est l'indométacine, l'ibuprofène ou le diclofénac.

4. Utilisation selon la revendication 1, dans laquelle l'anti-inflammatoire associé à un phospholipide est administré par voie intraveineuse.

5. Utilisation selon la revendication 1, dans laquelle l'anti-inflammatoire est un anti-inflammatoire non stéroïdien (AINS) et l'administration de l'AINS est réalisée par voie intraveineuse.

6. Utilisation d'une quantité efficace de la préparation parentérale d'un anti-inflammatoire associé à un phospholipide dans la préparation d'un médicament destiné à induire la fermeture du canal artériel.

7. Utilisation selon la revendication 6, dans laquelle le sujet est un nourrisson de faible poids de naissance.

8. Utilisation selon la revendication 6, dans laquelle l'anti-inflammatoire est l'indométacine, l'ibuprofène ou le diclofénac.

9. Utilisation selon la revendication 6, dans laquelle l'anti-inflammatoire associé à un phospholipide est administré par voie intraveineuse.

10. Utilisation selon la revendication 6, dans laquelle l'anti-inflammatoire est un anti-inflammatoire non stéroïdien (AINS) et l'administration de l'AINS est réalisée par voie intraveineuse.
